# EUROPÄISCHE PATENTANMELDUNG

(11) **EP 4 190 369 A1**
(43) Veröffentlichungstag der Anmeldung: **07.06.2023**
(21) Anmeldenummer: 21212099.2
(22) Anmeldetag: 02.12.2021
(51) Int. Cl.: A61M 1/00, A47L 9/14, B65F 1/00, B65F 1/06

(54) **VORRICHTUNG MIT EINEM BEHÄLTER UND EINEM ABSAUGBEUTEL FÜR EINE SAUGVORRICHTUNG UND MONTAGEVERFAHREN**

(71) Anmelder: BPR Swiss GmbH, 3672 Oberdiessbach (CH)
(72) Erfinder: MAURER, Marc, 3600 Thun (CH); MAURER, Marcel, 2560 Nidau (CH); STRAUCH, Patrick, 3512 Walkringen (CH)
(74) Vertreter: Rutz & Partner

(57) **Zusammenfassung**

Die Vorrichtung (1), die für eine Saugvorrichtung (8) im Bereich medizinischer oder industrieller Anwendungen vorgesehen ist, umfasst einen Behälter (2), der einen Behälterraum (200) mit einer daran anschliessenden Behälteröffnung (20) aufweist, eine Abdeckung (6) zum Schliessen der Behälteröffnung (20) und einen für den einmaligen Gebrauch vorgesehenen Absaugbeutel (3), der an einem Ende eine Beutelöffnung (30) aufweist und der im Behälterraum (200) angeordnet ist. Erfindungsgemäss ist innerhalb des Behälterraums (200) an dem der Behälteröffnung (20) zugewandten Ende des Behälters (2) wenigstens ein Stützelement (22) vorgesehen. Ferner ist ein Montagering (4) vorgesehen, der den Absaugbeutel (3) umschliesst und in ein einseitig geschlossenes Unterteil (31) und in ein mit der Beutelöffnung (30) versehenes Oberteil (32) aufteilt, welches Oberteil (32) über den Montagering (4) und einen Teil des Unterteils (31) zurückgefaltet ist, sodass der auf das wenigstens eine Stützelement (22) abgestützte Montagering (4) eine Aufnahmeöffnung (300) des Absaugbeutels (3) begrenzt.

## Beschreibung

Die Erfindung betrifft eine Vorrichtung mit einem Behälter und einem für den einmaligen Gebrauch vorgesehenen Absaugbeutel für eine Saugvorrichtung, die für medizinische oder industrielle Anwendungen vorgesehen ist, sowie ein Verfahren zur Montage des Absaugbeutels im Behälter.

Saugvorrichtungen werden in zahlreichen Bereichen der Industrie und der Medizin eingesetzt. Im Bereich der Dentalmedizin dienen Saugvorrichtungen der Erzeugung eines Unterdrucks an einer Absaugkanüle oder Absaugleitung, durch die Flüssigkeit und Feststoffpartikel, insbesondere Zahnschmelzpartikel, aus dem Mund eines Patienten abgesaugt werden.

Eine Saugvorrichtung dieser Art ist beispielsweise aus der EP3172439B1 bekannt. Diese Saugvorrichtung umfasst ein Pumpensystem, welches über eine Anschlussleitung und eine Medientrennvorrichtung, die einen Behälter umfasst, mit einer Absaugleitung verbunden ist. Die Anschlussleitung und die Absaugleitung sind mit der Abdeckung des Behälters verbunden, in dem die angesaugte Flüssigkeit von der Luft getrennt und gespeichert wird. Die Luft wird über die Anschlussleitung weiter zur Saugvorrichtung gefördert.

Der Behälter umfasst eine Überlaufsicherung, die verhindert, dass die Flüssigkeit bei steigendem Pegel zur Absaugleitung gelangen kann. Sobald der Pegel der Flüssigkeit den maximal zulässigen Wert erreicht hat, wird die Absaugleitung innerhalb des Behälters verschlossen.

Sobald der Behälter gefüllt ist, wird der Verschluss oder die Abdeckung des Behälters entfernt und die Flüssigkeit entsorgt.

Der Behälter wird mit entsprechendem Aufwand gereinigt, wobei darauf zu achten ist, dass medizinisches Personal nicht in Kontakt mit der Flüssigkeit gerät.

Bevorzugt wird im Behälter daher ein Absaugbeutel vorgesehen, der mit der angesaugten Flüssigkeit gefüllt und anschliessend verschlossen und entsorgt wird. Vorzugsweise werden auffaltbare oder flexible Absaugbeutel verwendet, die in hoher Anzahl mit minimalem Platzbedarf gelagert werden können. Die Abmessungen des aufgefalteten Absaugbeutels, die aus einer Kunststofffolie gefertigt werden, entsprechen zumindest annähernd den Abmessungen des Behälters.

Die WO200793670A1 offenbart einen Behälter mit einem Absaugbeutel für eine Saugvorrichtung, die für medizinische Einsätze vorgesehen ist. Der Behälter ist mit einer Abdeckung abschliessbar, an der der Absaugbeutel befestigt wird. Als Nachteil solcher Vorrichtungen wird angegeben, dass der Absaugbeutel bei der Verbindung der Abdeckung mit dem Behälter zwischen den oberen Rand des Behälters und die Abdeckung geraten kann, weshalb sich der Absaugbeutel möglicherweise nicht richtig entfalten kann und die Abdeckung den Behälter möglicherweise nicht dicht abschliesst. Weiter wird darauf hingewiesen, dass eine fehlerhafte Montage des Absaugbeutels insbesondere bei medizinischen Notfällen leicht auftreten kann. Zur Vermeidung dieses Problems werden Befestigungsmittel vorgesehen, welche den Absaugbeutel im gefalteten Zustand halten, bis dieser in den Behälter eingesetzt ist. Nach dem Einsetzen des Absaugbeutels muss hingegen sichergestellt werden, dass das Befestigungsmittel die Auffaltung des Absaugbeutels nicht länger behindert.

Typischerweise ist der Absaugbeutel mit der Abdeckung fest verbunden und wird zusammen mit der Abdeckung entsorgt, weshalb ein hoher Materialverbrauch und ein hoher Kostenaufwand zur Herstellung des Absaugbeutels resultieren. Weiter resultiert ein hoher Aufwand zur Entsorgung der Absaugbeutel, da die massive Abdeckung aus Plastik zu entsorgen ist.

Sofern der Absaugbeutel hingegen vom Anwender mit der Abdeckung verbunden wird, ist dies nur mit Geschick möglich. Der Saugbeutel muss präzise an ein Halteelement der Abdeckung angepasst sein und gegebenenfalls anhand eines Montagemittels, wie eines elastischen Rings, montiert werden.

Falls der gefüllte Absaugbeutel nicht zusammen mit der Abdeckung entsorgt wird, muss er nach dem Füllvorgang vorsichtig von der Abdeckung gelöst werden, um zu verhindern, dass Flüssigkeit freigesetzt wird. Dabei besteht die Gefahr, dass sich der gefüllte Absaugbeutel selbsttätig von der Abdeckung löst und die Flüssigkeit freigesetzt wird.

Der Absaugbeutel mit dem zusätzlichen Befestigungsmittel ist daher relativ aufwendig gestaltet und nur mit Geschick montierbar und demontierbar.

Zu beachten ist, dass bei der Montage dieser bekannten Absaugbeutel die Abdeckung manuell oder durch Saugdruck an den Behälter angepresst wird, weshalb zwischen dem Behälter und der Abdeckung normalerweise keine absolut dichte Verbindung resultiert.

Die WO200793670A1 offenbart ferner, dass im Raum zwischen dem Behälter und dem Absaugbeutel ein Unterdruck erzeugt wird, damit sich der Absaugbeutel entfalten kann und durch den Unterdruck, der innerhalb des Absaugbeutels vorherrscht, zusammengezogen wird.

Der vorliegenden Erfindung liegt daher die Aufgabe zugrunde, eine verbesserte Vorrichtung mit einem Behälter und einem für den einmaligen Gebrauch vorgesehenen Absaugbeutel für eine Saugvorrichtung zu schaffen, die bei medizinischen und industriellen Anwendungen einsetzbar ist. Weiterhin ist ein verbessertes Montagefahren anzugeben, mittels dessen der Absaugbeutel vorteilhaft im Behälter der Vorrichtung montierbar ist.

Der Saugbeutel soll einfach ausgestaltet und mittels des Montageverfahrens in einfacher Weise im Behälter montierbar und nach dem Füllen mit abgesagter Flüssigkeit wieder demontierbar sein. Insbesondere soll es nicht erforderlich sein, den Absaugbeutel mit Geschick zu montieren und mit Geschick zu demontieren, um zu verhindern, dass nach der Montage undichte Stellen resultieren und sich der Absaugbeutel bei der Demontage möglicherweise selbsttätig löst und die abgesaugte Flüssigkeit freigesetzt wird. Fehlmanipulationen sollen bei der Montage und Demontage des Absaugbeutels somit zuverlässig vermieden werden.

Auf besondere Montagemittel, die den Absaugbeutel gefaltet halten, bevor er montiert wird, und die zu lösen sind, bevor der Absaugbeutel gefüllt wird, soll verzichtet werden können. Dadurch soll auch vermieden werden, dass der Absaugbeutel teilweise gefaltet werden muss bevor er montiert wird und daher entsprechend viel Raum in Anspruch nimmt.

Weiterhin soll darauf verzichtet werden können, innerhalb des Behälters zwischen dem Absaugbeutel und der Wand des Behälters einen Unterdruck zu erzeugen.

Die erfindungsgemässe Vorrichtung soll auch bei hohen Ansaugleistungen von beispielsweise 100 l/min - 300 l/min einwandfrei funktionieren. Insbesondere soll es auch bei hohen Ansaugleistungen nicht notwendig sein, einen Unterdruck zwischen dem Absaugbeutel und dem Behälter vorzusehen.

Diese Aufgabe wird mit einer Vorrichtung mit einem Behälter und einem Absaugbeutel gemäss Anspruch 1, einem Absaugbeutel gemäss Anspruch 10 und einem Montageverfahren gemäss Anspruch 11 gelöst. Vorteilhafte Ausgestaltungen der Erfindung sind in weiteren Ansprüchen angegeben.

Die Vorrichtung, die für eine Saugvorrichtung im Bereich medizinischer oder industrieller Anwendungen vorgesehen ist, umfasst einen Behälter, der einen Behälterraum mit einer daran anschliessenden Behälteröffnung aufweist, eine Abdeckung zum Schliessen der Behälteröffnung und einen für den einmaligen Gebrauch vorgesehenen Absaugbeutel, der an einem Ende eine Beutelöffnung aufweist und der im Behälterraum angeordnet ist bzw. wird.

Erfindungsgemäss ist innerhalb des Behälterraums an dem der Behälteröffnung zugewandten Ende des Behälters wenigstens ein Stützelement vorgesehen, das vorzugsweise in den Behälterraum hineinragt. Ferner ist ein Montagering vorgesehen, der den Absaugbeutel umschliesst und in ein einseitig geschlossenes Unterteil und in ein mit der Beutelöffnung versehenes Oberteil aufteilt, welches Oberteil über den Montagering und einen Teil des Unterteils zurückgefaltet ist, sodass der auf das wenigstens eine Stützelement abgestützte Montagering eine Aufnahmeöffnung des vorzugsweise aus einer gemäss dem Plastikfolie gefertigten Absaugbeutels begrenzt.

Gemäss dem Verfahren zur Montage des Absaugbeutels in der Vorrichtung ist vorgesehen
- dass in einem ersten Montageschritt der Montagering über den Absaugbeutel geführt wird, sodass der Montagering den Absaugbeutel umschliesst und in das einseitig geschlossene Unterteil und in das mit der Beutelöffnung versehene Oberteil aufteilt,
- dass in einem zweiten Montageschritt das Oberteil radial nach aussen über den Montagering und einen Teil des Unterteils zurückgefaltet wird, sodass der Montagering eine Aufnahmeöffnung des Absaugbeutels begrenzt,
   dass in einem dritten Montageschritt der Absaugbeutel in den Behälterraum des geöffneten Behälters eingeführt und der Montagering auf das wenigstens eine Stützelement abgestützt wird, und
- dass in einem vierten Montageschritt die Abdeckung oder ein mit der Abdeckung verbundener Anschlussring auf den Behälter aufgesetzt wird.

Sofern dies nicht bereits geschehen ist, wird die Vorrichtung durch eine an die Abdeckung angeschlossene Anschlussleitung mit der Saugvorrichtung verbunden und die Saugvorrichtung in Betrieb gesetzt. Über eine Absaugleitung, die mit der Abdeckung verbunden ist, kann in der Folge Flüssigkeit in den Absaugbeutel transferiert werden, wobei angesaugte Luft und Flüssigkeit innerhalb des Behälters voneinander getrennt werden. Die angesaugte Luft wird über die Anschlussleitung weiter zur Saugvorrichtung geführt, während die Flüssigkeit im Behälter gespeichert wird.

Nach Abschluss des Saugvorganges wird die Abdeckung oder der mit der Abdeckung verbundene Anschlussring vom Behälter gelöst und der Montagering erfasst und mit dem Absaugbeutel aus dem Behälter entfernt. Nach dem Lösen der Abdeckung ist der Montagering frei zugänglich, sodass er bequem erfasst und angehoben werden kann.

Der Absaugbeutel umfasst vorzugsweise eine Zugleine, die nach der Entfernung des mit Flüssigkeit gefüllten Absaugbeutels aus dem Behälter betätigt wird, um die Beutelöffnung dicht abzuschliessen. Die Zugleine ist beispielsweise in Ösen oder in einem schlauchförmigen Abschlussstück des Absaugbeutels gehalten, das gegebenenfalls die Beutelöffnung begrenzt.

Der verschlossene Absaugbeutel kann nun nach Entfernen des Montagerings oder zusammen mit dem Montagering sicher entsorgt werden. Sofern der Montagering entfernt wird, kann er in der Folge mit einem neuen Absaugbeutel verbunden werden.

Die Sicherheit in der Handhabung des Absaugbeutels kann weiter erhöht werden, indem Flüssigkeit absorbierendes Material im Absaugbeutel vorgesehen wird.

Der Vorgang zur Montage und Demontage des Absaugbeutels kann mit einfachen Massnahmen durchgeführt werden, ohne dass sich der Absaugbeutel selbsttätig lösen und Flüssigkeit aus dem Beutel austreten kann.

Zur Montage des Absaugbeutels muss dieser nicht mit der Abdeckung verbunden werden. Eine Verbindung mit der Abdeckung verursacht Schwierigkeiten, weil der Absaugbeutel dicht abschliessend mit der Abdeckung zu verbinden und dabei zu beachten ist, dass sich der Absaugbeutel nicht wieder selbsttätig löst. Stattdessen wird das mit der Beutelöffnung versehene Endstück des Absaugbeutels durch den Montagering hindurch geführt und radial nach allen Seiten über den Montagering hinweg und zurückgeführt. Bei diesem Vorgang ist lediglich der Montagering und nicht die gesamte Abdeckung zu manipulieren. Da das Endstück des Absaugbeutels in einfacher Weise radial nach aussen um den Montagering herumgeführt wird resultiert praktisch automatisch eine zuverlässige Verbindung zwischen dem Montagering und dem Absaugbeutel.

Der mit dem Montagering versehene Absaugbeutel kann nun in einfacher Weise in den Behälter eingesetzt werden, ohne dass die Abdeckung, die normalerweise mit Leitungen verbunden ist, störend in Erscheinung tritt. Erst nachdem der Absaugbeutel bequem in den Behälter eingesetzt und der Montagering auf das wenigstens eine Stützelement aufgesetzt wurde, wird der Behälter mit der Abdeckung verschlossen. Dabei werden die eingangs beschriebenen Probleme aus dem Stand der Technik vermieden, wonach der mit der Abdeckung verbundene Absaugbeutel zwischen der Abdeckung und dem Behälter festgeklemmt werden kann. Stattdessen wurde der Absaugbeutel vollständig in den Behälter eingesetzt, sodass die Abdeckung ohne jegliche Schwierigkeiten aufgesetzt werden kann und ein dichter Abschluss zwischen dem Behälter und der Abdeckung oder zwischen dem Behälter und dem mit der Abdeckung verschraubten Anschlussring resultiert.

Nach Abschluss des Saugvorgangs kann die Abdeckung wieder bequem und ohne besondere Vorsicht entfernt werden, da der Absaugbeutel nicht mit der Abdeckung verbunden ist. Es besteht somit auch keine Gefahr, dass sich der Absaugbeutel während der Entnahme durch mögliche Krafteinwirkungen von der Abdeckung löst. Stattdessen kann der Montagering erfasst und mit dem Absaugbeutel aus dem geöffneten Behälter entfernt werden. Das über den Montagering geführte Oberteil des Absaugbeutels kann nun wieder zurückgeführt und durch Betätigung der Zugleine verschlossen werden. Bei diesen Vorgang wird der Montagering wieder freigelegt, sodass er bequem entfernt und wieder verwendet werden kann. Da das Oberteil des Beutels um den Montagering herumgeführt wurde, wurde auch eine Kontaktierung des Montagerings mit der angesaugten Flüssigkeit vermieden. Der Montagering ist daher nicht kontaminiert.

Die beschriebenen Montageschritte können alle vom Anwender in einfacher Weise durchgeführt werden. Die Montageschritte zur Verbindung des Montagerings mit dem Absaugbeutel können jedoch auch auf Seiten des Herstellers durchgeführt werden, sodass dem Anwender Absaugbeutel zur Verfügung gestellt werden, die bereits einen Montagering aufweisen und direkt in den Behälter eingesetzt werden können. In diesem Fall ist nur ein dünner Montagering und nicht, wie bei den eingangs beschriebenen Lösungen, eine gesamte Abdeckung mit dem Absaugbeutel zu entsorgen.

Der Montagering ist vorzugsweise elastisch deformierbar und/oder aus einem hartelastischen oder weichelastischen Material gefertigt. Die Elastizität des Montagerings erlaubt es, diesen in einfacher Weise zu montieren.

Das wenigstens eine Stützelement ist vorzugsweise als umlaufende Stufe oder Schulter an der Innenseite des aus Plastik gefertigten Behälters angeformt oder als umlaufende Nut in die Wand des Behälters eingesenkt, wobei die Unterseite der Nut vorzugsweise eine Schulter bildet. Das wenigstens eine Stützelement kann daher vorteilhaft einstückig an der Innenseite der Behälterwand angeformt sein.

Der Montagering kann daher mit einem Handgriff auf die Schulter aufgesetzt und/oder in die umlaufende Nut eingesetzt werden, in der der elastische Montagering gehalten ist und erst durch Krafteinwirkung wieder gelöst wird. Aufgrund der Elastizität des Montagerings ist dies problemlos möglich.

Besonders vorteilhaft ist die Anordnung des Montagerings auf der Schulter, durch die der Montagering fest gehalten wird und mittels der Abdeckung oder des Anschlussrings gegebenenfalls komprimiert und vorzugsweise dicht abschliessend gegen die Schulter gedrückt werden kann.

In einer weiteren vorzugsweisen Ausgestaltung ist vorgesehen, dass die Abdeckung oder der mit der Abdeckung verbundene Anschlussring einen Flanschring aufweist, mittels dessen ein Dichtungsring dicht abschliessend gegen eine ringförmig verlaufende Oberkante des Behälters gedrückt wird.

Zur Montage der Abdeckung ist die Innenwand des Behälters zwischen der Behälteröffnung und dem wenigstens einen Stützelement vorzugsweise mit einem Innengewinde versehen, auf das die Abdeckung oder der mit der Abdeckung verschraubte Anschlussring aufgeschraubt werden kann.

Der in einer vorzugsweisen Ausgestaltung vorgesehene Anschlussring weist vorzugsweise auf einer Seite ein mit dem Behälter verschraubbares erstes Gewindeteil und auf der anderen Seite ein mit der Abdeckung verschraubbares zweites Gewindeteil sowie einen aussen liegenden ringförmigen Flanschring auf, durch den der Dichtungsring dicht abschliessend gegen die Oberkante des Behälters gedrückt wird.

Durch die durch Gewindeelemente realisierte Verbindung der Abdeckung gegebenenfalls des Anschlussrings mit der Abdeckung mit dem Behälter resultiert eine stabile und bereits relativ dicht abgeschlossene Verbindung zwischen dem Behälter und der Abdeckung.

Der Behälter wird zudem mittels des Montagerings, der gegen die ringförmig verlaufende Schulter innerhalb des Behälters gedrückt wird, und/oder mittels des Dichtungsrings, der gegen die ringförmig verlaufende Oberkante des Behälters gedrückt wird, dicht abgeschlossen.

Es hat sich gezeigt, dass der Absaugbeutel bei diesem dichtem Abschluss des Behälters und auch bei hohen Saugleistungen nicht gegen die Abdeckung gezogen wird und sich frei entfalten kann. Es ist daher nicht notwendig, zwischen der Aussenseite des Absaugbeutels und der Innenseite des Behälters einen Unterdruck zu erzeugen. Ein solcher Unterdruck wird mittels einer Hilfsleitung, die mit der Anschlussleitung oder direkt mit der Saugvorrichtung verbunden ist, nur optional vorgesehen.

Nachfolgend wird die Erfindung anhand von Zeichnungen näher erläutert. Dabei zeigt:
- Fig. 1a: eine erfindungsgemässe Vorrichtung 1 mit einem Behälter 2 und einem darin angeordneten Absaugbeutel 3 (schematisch gezeigt) sowie mit einer Abdeckung 6, die durch einen Anschlussring 5 mit dem Behälter 2, durch eine Anschlussleitung 82 mit einer Saugvorrichtung 8 und durch eine Absaugleitung 81 mit einem Saugkopf 811 verbunden ist;
- Fig. 1b: die Vorrichtung 1 von Fig. 1 mit dem Absaugbeutel 3, dem Behälter 2 und dem Anschlussring 5 in einem Längsschnitt mit Blick auf eine Überlaufsicherung 91, die innerhalb des Behälters 2 den Pegelstand der Flüssigkeit misst;
- Fig. 2: einen Teil der Vorrichtung 1 von Fig. 1 mit einem Teil des Absaugbeutels 3, der innerhalb des Behälters 2 von einem Montagering 4 gehalten ist;
- Fig. 3: die Abdeckung 6, den Anschlussring 5 und einen Teil des Behälters 2 von Fig. 2 voneinander getrennt in Schnittdarstellung;
- Fig. 4a: den Absaugbeutel 3 von Fig. 1 in einer vorzugsweisen Ausgestaltung bei einem ersten Schritt des Montageverfahrens, bei dem dem Absaugbeutel 3 der Montagering 4 übergestreift wird, der den Absaugbeutel 3 in einen abgeschlossenen unteren Teil 31 und einen mit der Beutelöffnung 20 versehene oberen Teil 32 unterteilt; und
- Fig. 4b: den Absaugbeutel 3 von Fig. 4a bei einem zweiten Schritt des Montageverfahrens, bei dem der obere Teil 32 des Absaugbeutels 3 über den Montagering 4 nach unten geführt wird, wonach der Absaugbeutel 3 am Montagering 4 erfasst und in den Behälter 2 eingesetzt werden kann.

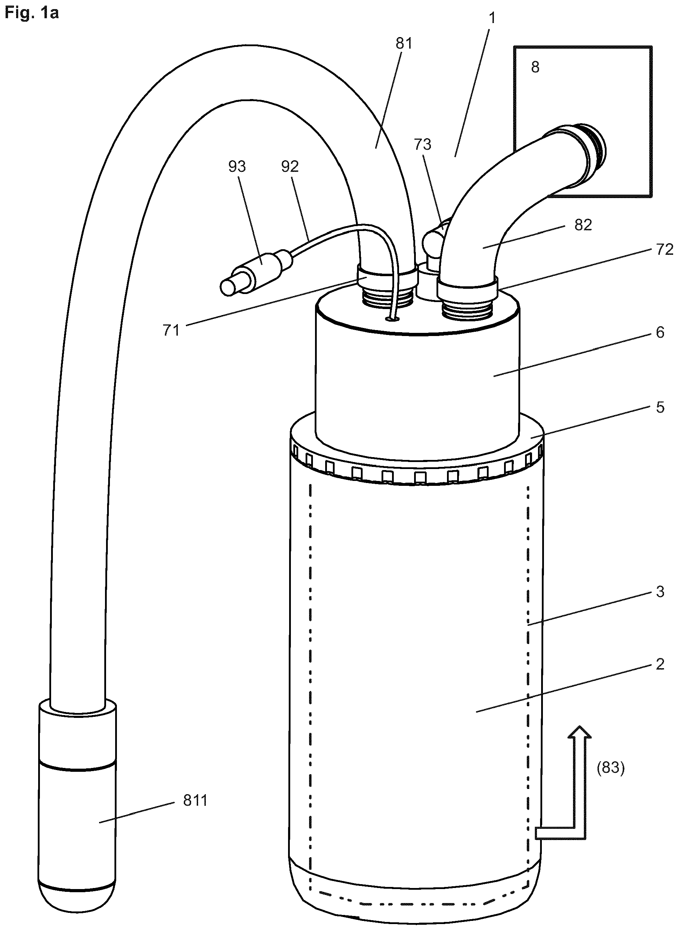

Fig. 1a zeigt eine erfindungsgemässe Vorrichtung 1 mit einem Behälter 2 und einem darin angeordneten Absaugbeutel 3 (strichpunktiert dargestellt). Ferner ist eine Abdeckung 6 vorgesehen, die optional durch einen Anschlussring 5 mit dem Behälter 2 verbunden ist. Alternativ kann die Abdeckung 6 auch direkt auf den Behälter 2 aufgesetzt werden. Dies ist beispielsweise möglich, indem der Anschlussring 5 einstückig an der Abdeckung 6 angeformt und nicht durch ein Gewinde mit der Abdeckung 6 verbunden wird. Durch die Wahl eines entsprechenden Anschlussrings 5 können nach Wunsch unterschiedliche Abdeckungen 6 auf den Behälter 2 aufgesetzt werden.

Der Behälter 2 und die Abdeckung sind vorzugsweise aus Kunststoff gefertigt. Der Anschlussring 5 ist vorzugsweise aus Metall gefertigt.

Die Abdeckung 6 ist durch eine Anschlussleitung 82 mit einer Saugvorrichtung 8 und durch eine Absaugleitung 81 mit einem Saugkopf 811 verbunden. Die Anschlussleitung 82 und die Absaugleitung 81 sind mit Anschlusselementen 71, 72 verbunden, die in Gewindebohrungen der Abdeckung 6 angeschraubt sind.

Mit der Abdeckung 6 ist ein Halteelement 73 verbunden, mittels dessen die Vorrichtung 1 beispielsweise mit der Saugvorrichtung 8 verbunden werden kann.

Weiterhin ist eine optionale Hilfsleitung 83 gezeigt, die direkt oder indirekt mit der Saugvorrichtung 8 verbunden wird und über die ein Unterdruck innerhalb des Behälters 2 zwischen der Aussenseite des Absaugbeutels 3 und der Innenwand des Behälters 2 erzeugt werden kann. Durch die erfindungsgemässe Abdichtung des Behälters 2 kann auf diese Hilfsleitung 83 jedoch verzichtet werden.

Aus der Abdeckung 6 ist zudem ein Messkabel 92 herausgeführt, dass innerhalb des Behälters mit einer Pegelmessvorrichtung oder Überlaufsicherung und ausserhalb des Behälters 2 mit einem Anschlussstecker 93 verbunden ist. Das Messkabel 92 wird mit einer Steuereinheit verbunden, welche die Saugvorrichtung 8 steuert und bei Feststellung eines Überlaufs im Behälter 2 ein optisches oder akustisches Warnsignal generiert oder die Saugvorrichtung 8 abschaltet.

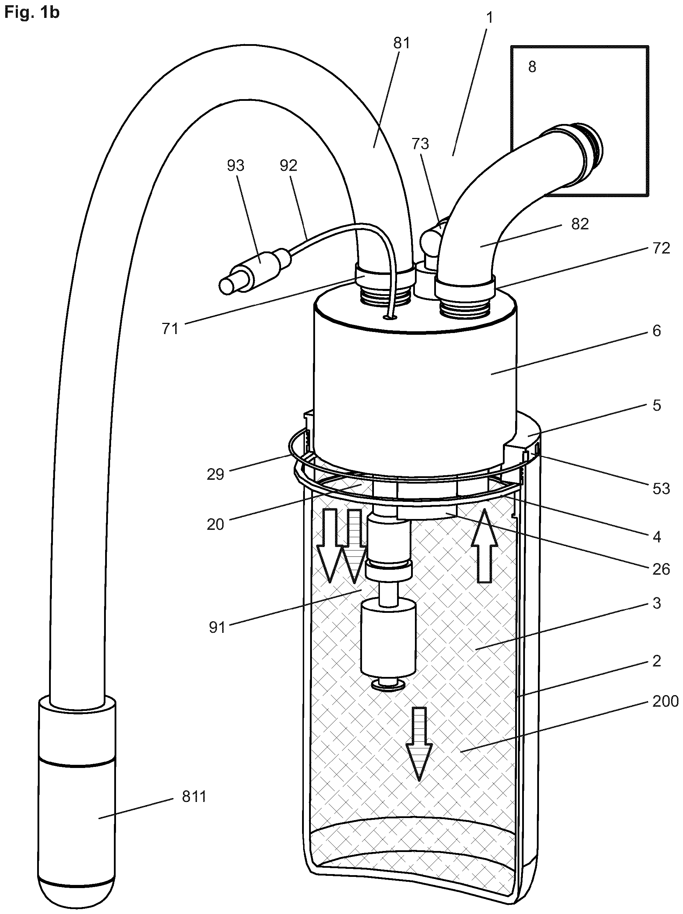

Fig. 1b zeigt die Vorrichtung 1 von Fig. 1 mit dem Absaugbeutel 3, dem Behälter 2 und dem Anschlussring 5 in einem Längsschnitt mit Blick auf die Überlaufsicherung 91, die im Innenraum bzw. Behälterraum 200 des Behälters 2 angeordnet ist und den Pegelstand der Flüssigkeit misst.

Der Anschlussring 5 weist einen Aussenflansch 53 auf, durch den ein Dichtungsring 29 dicht abschliessend mit einer Oberkante des Behälters 2 verbunden wird. Nach dem Aufsetzen der Abdeckung 6 oder der Abdeckung mit dem Anschlussrings 5 ist der Behälter 2 daher dicht abgeschlossen.

Der Behälter 2 weist nahe an der Behälteröffnung 20 ein Innengewinde 21 und unterhalb des Innengewindes 21 eine Stützelement 22 in der Form einer umlaufenden und vorzugsweise in sich geschlossenen Schulter auf. Auf das Stützelement 22 ist ein Montagering 4 abgestützt, von dem der schraffiert gezeigte Absaugbeutel 3 gehalten ist.

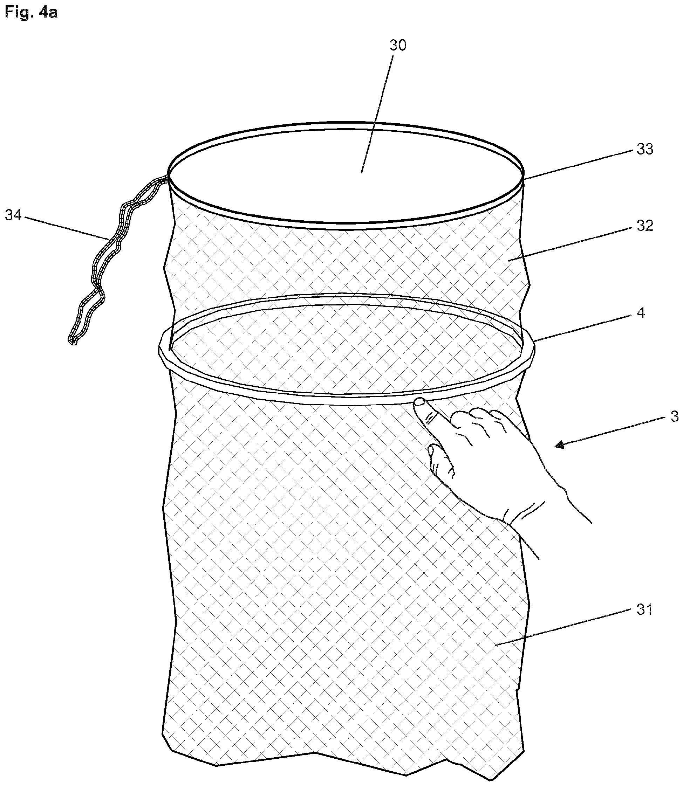

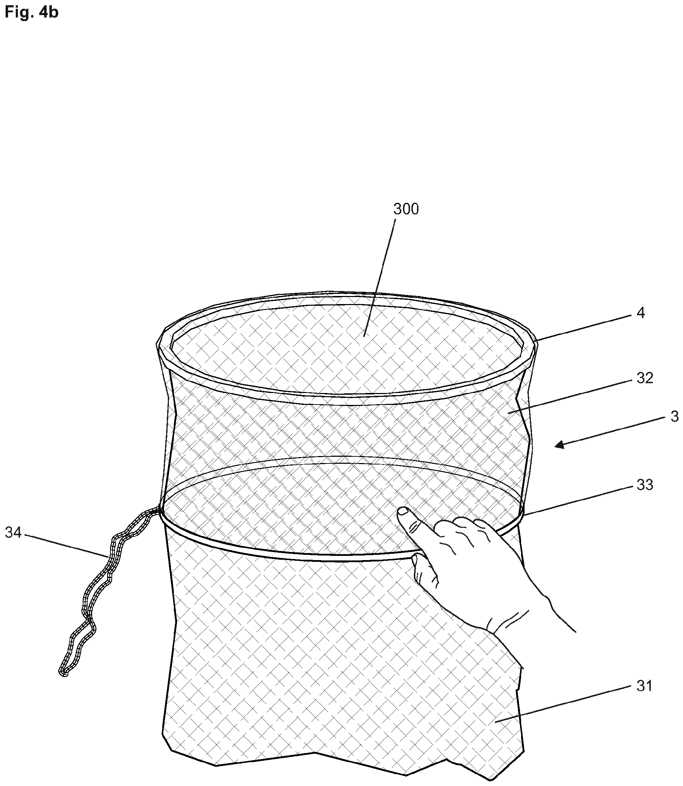

Der Absaugbeutel 3 und die Schritte zur Montage des Absaugbeutels 3 im Behälter 2 sind in Fig. 4a und Fig. 4b illustriert.

Die für den einmaligen Gebrauch vorgesehenen Absaugbeutel 3 sind aus einer Plastikfolie gefertigt, an der Unterseite verschlossen und an der Oberseite mit einer Beutelöffnung 30 versehen. Vorzugsweise anschliessend an die Beutelöffnung 30 ist wenigstens ein Haltemittel 33, wie Schlaufen, Ösen, Schlauchelemente, Rohrelemente oder ein Saum, vorgesehen, von dem ein Zugmittel 34 gehalten ist, mittels dessen die Beutelöffnung 30 abschliessbar ist. Bevorzugt wird ein Saum 33 vorgesehen, der gebildet wird, indem angrenzend an die Beutelöffnung 30 ein Endstück des Absaugbeutels 3 nach aussen gefaltet und mit der Plastikfolie verschweisst wird.

Absaugbeutel 3 werden vorzugsweise in einer Verpackung geliefert, in der eine hohe Anzahl von Absaugbeuteln 3 zugefaltet flächig aufeinander liegen.

Fig. 4a zeigt, dass in einem ersten Montageschritt der Montagering 4 über den Absaugbeutel 3 geführt wurde, sodass der Montagering 4 den Absaugbeutel 3 umschliesst und in ein einseitig geschlossenes Unterteil 31 und in ein mit der Beutelöffnung 30 versehenes Oberteil 32 aufteilt. Zur Vorbereitung dieses Montageschritts wird der Absaugbeutel 3 vorzugsweise aufgefaltet, so dass er beispielsweise eine zylindrische oder schlauchförmige Form annimmt. Der Montagering 4 kann weit nach oben geführt werden, so dass das Oberteil 32 vorzugsweise wesentlich kürzer ist als dargestellt.

Fig. 4b zeigt, dass in einem zweiten Montageschritt das Oberteil 32 radial nach aussen über den Montagering 4 und einen Teil des Unterteils 31 zurückgefaltet wird, sodass der Montagering 4 eine Aufnahmeöffnung 300 des Absaugbeutels 3 begrenzt.

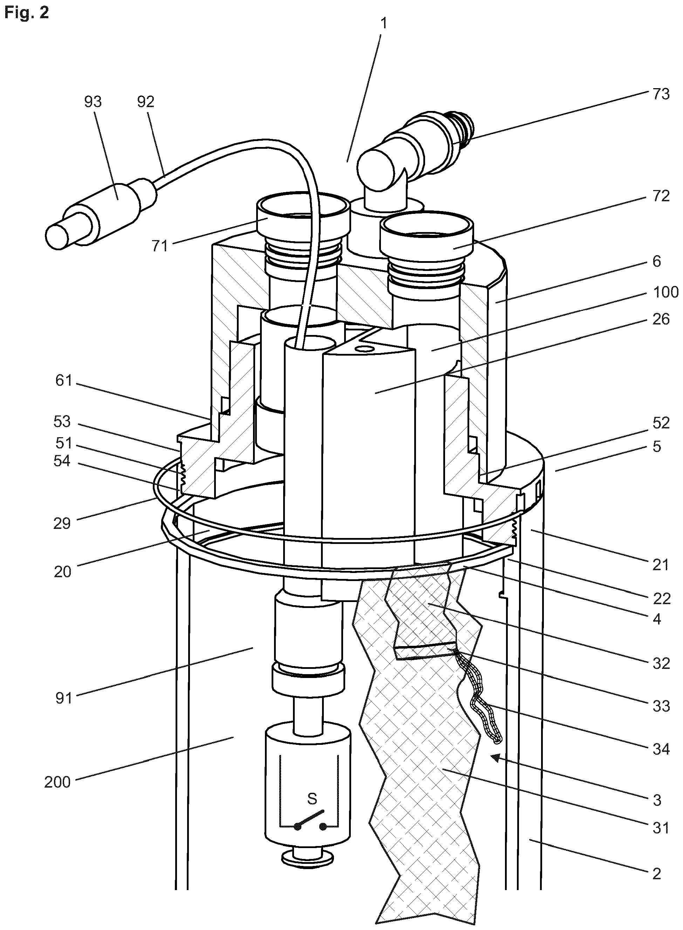

In einem dritten Montageschritt wird der mit dem Montagering 4 verbundene Absaugbeutel 3 in den Behälterraum 200 des geöffneten Behälters 2 eingeführt und der Montagering 4 auf das wenigstens eine Stützelement 22 abgestützt, wie dies in Fig. 2 gezeigt ist.

In einem vierten Montageschritt die Abdeckung 6 bzw. der mit der Abdeckung 6 verbundene Anschlussring 5 auf den Behälter 2 aufgesetzt.

Alle Montageschritte können bequem ausgeführt werden, ohne den Behälter 2 oder die Abdeckung 6 zu manipulieren.

Fig. 2 zeigt, dass der Absaugbeutel 3 am Montagering 4 aufgehängt ist und das fast vollständig um den Montagering 4 herum geführte Oberteil 32 des Absaugbeutels 3 mit der Zugleine 34 nach unten hängt. Dabei liegt nicht der Montagering 4, sondern das Oberteil 32 des Absaugbeutels 3 am schulterförmigen Stützelement 22 und an der Unterseite des Anschlussrings 5 an. Der Montagering 4 ist daher isoliert und kann von der angesaugten Flüssigkeit nicht kontaminiert werden.

Sofern der Montagering 4 aus weichelastischem Material gefertigt ist, wird er durch die Abdeckung 6 bzw. den Anschlussring 5 vorzugsweise leicht komprimiert, so dass auch der Montagering 4 einen Dichtungsring bildet. Auf den Dichtungsring 29 kann daher verzichtet werden, wenn mit dem Montagering 4 eine genügende Abdichtung des Behälters 2 erzielt wird.

Die Abdeckung 6 ist dicht abgeschlossen mit dem Anschlussring 5 verbunden, der mit dem Behälter 2 verschraubt ist und mit dem Flanschring 53 den Dichtungsring 29 an die Oberkante des Behälters 2 andrückt. Nach dem Aufsetzen der Abdeckung 6 und des optional vorgesehenen Anschlussrings 5 ist der Behälter 2 dicht abgeschlossen.

Fig. 2 zeigt, dass das an die Behälteröffnung 20 anschliessende Innengewinde 21 mit einem ersten Gewindeteil 51 an der Unterseite des Anschlussrings 5 verschraubt ist. Das untere Ende 54 des ersten Gewindeteils 51 liegt vorzugsweise am Montagering 4 an. Das zweite Gewindeteil 52 an der Oberseite des Anschlussrings 5 ist mit einem Innengewinde eines Anschlussflanschs 61 der Abdeckung 6 verschraubt.

Nach der Montage des Absaugbeutels 3 kann die Absaugvorrichtung in Betrieb genommen werden. Durch die Saugvorrichtung 8 wird über die Anschlussleitung 82 im Behälter 2 ein Unterdruck erzeugt, der durch die Absaugleitung 81 auf den Saugkopf 811 übertragen wird. Bei einem medizinischen Einsatz, z.B. in einer Zahnarztpraxis, werden über den Saugkopf 811 Luft und Flüssigkeit des Patienten angesaugt, wie dies in Fig. 1b mit einem weissen Pfeil und einem schraffierten Pfeil symbolisiert ist.

Die Flüssigkeit (schraffierter Pfeil) wird im Behälter 2 gespeichert, während Luft (weisser Pfeil) um ein Abscheideelement 26 herum und über die Anschlussleitung 82 zur Saugvorrichtung 8 geführt wird.

Fig. 2 zeigt weiterhin, dass die Überlaufsicherung 91 vorzugsweise einen Schwimmer und einen Sensor oder einen Schalter S aufweist, welcher beim Überlauf des Behälters 2 durch die Verschiebung des Schwimmers aktiviert oder betätigt wird. Die Betätigung des Schalters oder Sensors wird von einer Steuereinheit erfasst, welche die Saugvorrichtung 8 entsprechend steuert.

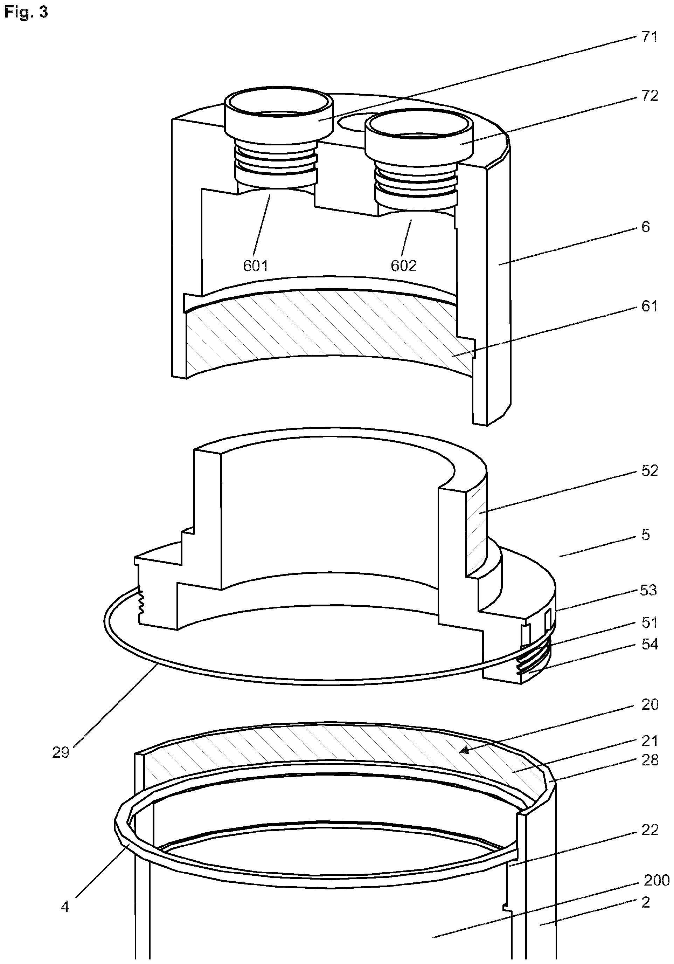

Fig. 3 zeigt die Abdeckung 6 mit den beiden Abschlusselementen 71, 72, die in Gewindebohrungen 601, 602 der Abdeckung 6 eingesetzt sind, den Anschlussring 5 mit dem Dichtungsring 29 und einen Teil des Behälters 2 mit dem Montagering 4 von Fig. 2 voneinander getrennt und in Schnittdarstellung.

Der Montagering 4 ist auf das schulterförmige Stützelement 22 aufgesetzt, das in den Behälterraum 200 hineinragt. Zwischen der Behälteröffnung 20 und den Stützelement 22 ist das schraffiert gezeigte Innengewinde vorgesehen. In das Innengewinde 21 des Behälters 2 wird das mit einem Aussengewinde versehene erste Gewindeteil 51 des Anschlussrings 5 eingeschraubt. Das zweite Gewindeteil 52 des Anschlussrings 5 weist ein schraffiert gezeigtes Aussengewinde auf, welches zu einem schraffiert gezeigten Innengewinde 61 der Abdeckung 6 korrespondiert. Der Dichtungsring 29 liegt unterhalb des Aussenflanschs 53 des Anschlussrings 5 und wird von diesem gegen eine Oberkante 28 des Behälters 2 gedrückt, sobald der Anschlussrings 5 mit dem Behälter 2 verschraubt wird. Bei diesem Vorgang wird die Unterseite 54 des ersten Gewindeteils 51 des Anschlussrings 5 vorzugsweise so weit gegen den Montagering 4 gedreht, dass dieser fixiert und gegebenenfalls komprimiert wird.

Fig. 4a zeigt den Absaugbeutel 3 von Fig. 1 in einer vorzugsweisen Ausgestaltung bei einem ersten Schritt des oben beschriebenen Montageverfahrens.

Fig. 4b zeigt den Absaugbeutel 3 von Fig. 4a bei einem zweiten Schritt des Montageverfahrens.

## Patentansprüche

1. Vorrichtung (1) für eine Saugvorrichtung (8) mit einem Behälter (2), der einen Behälterraum (200) mit einer daran anschliessenden Behälteröffnung (20) aufweist, mit einer Abdeckung (6) zum Schliessen der Behälteröffnung (20) und mit einem für den einmaligen Gebrauch vorgesehenen Absaugbeutel (3), der an einem Ende eine Beutelöffnung (30) aufweist und der im Behälterraum (200) angeordnet ist, **dadurch gekennzeichnet, dass** innerhalb des Behälterraums (200) an dem der Behälteröffnung (20) zugewandten Ende des Behälters (2) wenigstens ein Stützelement (22) vorgesehen ist und dass ein Montagering (4) vorgesehen ist, der den Absaugbeutel (3) umschliesst und in ein einseitig geschlossenes Unterteil (31) und in ein mit der Beutelöffnung (30) versehenes Oberteil (32) aufteilt, welches Oberteil (32) über den Montagering (4) und einen Teil des Unterteils (31) zurückgefaltet ist, sodass der Montagering (4) eine Aufnahmeöffnung (300) des Absaugbeutels (3) begrenzt, und dass der Montagering (4) auf das wenigstens eine Stützelement (22) abgestützt ist.

2. Vorrichtung (1) nach Anspruch 1, **dadurch gekennzeichnet, dass** der Montagering (4) elastisch deformierbar ist und dass der Montagering (4) aus einem hartelastischen oder weichelastischen Material gefertigt ist.

3. Vorrichtung (1) nach Anspruch 1 oder 2, **dadurch gekennzeichnet, dass** das wenigstens eine Stützelement (22) als umlaufende Stufe oder Schulter an der Innenseite des aus Plastik gefertigten Behälters (2) angeformt ist oder dass das wenigstens eine Stützelement (22) eine umlaufende Nut ist, deren Unterseite vorzugsweise eine Schulter bildet.

4. Vorrichtung (1) nach einem der Ansprüche 1 - 3, **dadurch gekennzeichnet, dass** der Montagering (4) durch die Abdeckung (6) oder durch einen mit der Abdeckung (6) verbundenen Anschlussring (5) in Position gehalten oder vorzugsweise dicht abschliessend gegen das wenigstens eine Stützelement (22) gedrückt wird.

5. Vorrichtung (1) nach einem der Ansprüche 1 - 4, **dadurch gekennzeichnet, dass** die Abdeckung (6) oder der mit der Abdeckung (6) verbundene Anschlussring (5) einen Flanschring (53) aufweist, mittels dessen ein Dichtungsring (29) dicht abschliessend gegen eine ringförmig verlaufende Oberkante (28) des Behälters (2) gedrückt wird.

6. Vorrichtung (1) nach einem der Ansprüche 1 - 5, **dadurch gekennzeichnet, dass** der Absaugbeutel (3) eine Zugleine (34) umfasst, durch deren Betätigung der Absaugbeutel (3) oder die Beutelöffnung (30) abschliessbar ist, wobei die Zugleine (34) vorzugsweise in Ösen oder in einem schlauchförmigen Abschlussstück (33) des Absaugbeutels (3), das die Beutelöffnung (30) begrenzt, gehalten ist.

7. Vorrichtung (1) nach einem der Ansprüche 1 - 6, **dadurch gekennzeichnet, dass** zwischen der Behälteröffnung (20) und dem wenigstens einen Stützelement (22) ein Innengewinde (21) vorgesehen ist, auf das die Abdeckung (6) oder der mit der Abdeckung (6) verschraubte Anschlussring (5) aufgeschraubt ist.

8. Vorrichtung (1) nach Anspruch 7, **dadurch gekennzeichnet, dass** der Anschlussring (5) auf einer Seite ein mit dem Behälter (5) verschraubtes erstes Gewindeteil (51) und auf der anderen Seite ein mit der Abdeckung (6) verschraubtes zweites Gewindeteil (52) und einen aussen liegenden ringförmigen Flanschring (53) aufweist, durch den der Dichtungsring (29) dicht abschliessend gegen die Oberkante (28) des Behälters (2) gedrückt wird.

9. Vorrichtung (1) nach einem der Ansprüche 1 - 8, **dadurch gekennzeichnet, dass** die Abdeckung (6) durch Anschlusselemente (71, 72) mit einer Anschlussleitung (82), die an die Saugvorrichtung (8) angeschlossen ist, und mit einer Absaugleitung (81) verbunden ist.

10. Absaugbeutel (3) mit einer Beutelöffnung (30) für eine Vorrichtung (1) nach einem der Ansprüche 1 - 9, **dadurch gekennzeichnet, dass** ein Montagering (4) vorgesehen ist, der den Absaugbeutel (3) umschliesst und in ein einseitig geschlossenes Unterteil (31) und in ein mit der Beutelöffnung (30) versehenes Oberteil (32) aufteilt, welches Oberteil (32) über den Montagering (4) und einen Teil des Unterteils (31) zurückgefaltet ist, sodass der Montagering (4) eine Aufnahmeöffnung (300) des Absaugbeutels (3) begrenzt, und dass der Absaugbeutel (3) eine Zugleine (34) umfasst, durch deren Betätigung der Absaugbeutel (3) oder die Beutelöffnung (30) abschliessbar ist, wobei die Zugleine (34) vorzugsweise in Ösen oder in einem schlauchförmigen Abschlussstück (33) des Absaugbeutels (3), das die Beutelöffnung (30) begrenzt, gehalten ist.

11. Verfahren zur Montage eines für den einmaligen Gebrauch vorgesehenen Absaugbeutels (3), der an einem Ende eine Beutelöffnung (30) aufweist, in einem Behälter (2), einer Vorrichtung (1) nach einem der Ansprüche 1 - 9, welcher Behälter (2) einen Behälterraum (200) mit wenigstens einem darin vorgesehenen Stützelement (22), auf das ein Montagering (4) abstützbar ist, und mit einer daran anschliessenden Behälteröffnung (20) aufweist, die mit einer Abdeckung (6) abschliessbar ist, **dadurch gekennzeichnet,**
**dass** in einem ersten Montageschritt der Montagering (4) über den Absaugbeutel (3) geführt wird, sodass der Montagering (4) den Absaugbeutel (3) umschliesst und in ein einseitig geschlossenes Unterteil (31) und in ein mit der Beutelöffnung (30) versehenes Oberteil (32) aufteilt,
**dass** in einem zweiten Montageschritt das Oberteil (32) radial nach aussen über den Montagering (4) und einen Teil des Unterteils (31) zurückgefaltet wird, sodass der Montagering (4) eine Aufnahmeöffnung (300) des Absaugbeutels (3) begrenzt,
**dass** in einem dritten Montageschritt der Absaugbeutel (3) in den Behälterraum (200) des geöffneten Behälters (2) eingeführt und der Montagering (4) auf das wenigstens eine Stützelement (22) abgestützt wird, und
**dass** in einem vierten Montageschritt die Abdeckung (6) oder ein mit der Abdeckung (6) verbundener Anschlussring (5) auf den Behälter (2) aufgesetzt wird.

12. Verfahren nach Anspruch 11, **dadurch gekennzeichnet, dass** der Montagering (4) durch die Abdeckung (6) oder durch den Anschlussring (5), der mit der Abdeckung (6) verbunden ist oder verbunden wird, dicht abschliessend gegen das wenigstens eine Stützelement (22) gepresst wird.

13. Verfahren nach Anspruch 11 oder 12, **dadurch gekennzeichnet, dass** die Vorrichtung (1) durch eine an die Abdeckung (6) angeschlossene Anschlussleitung (82) mit einer Saugvorrichtung (8) verbunden wird, dass die Saugvorrichtung (8) in Betrieb gesetzt wird und dass über eine Absaugleitung (81), die mit der Abdeckung (6) verbunden ist, Flüssigkeit in den Absaugbeutel (3) transferiert wird, wonach die Abdeckung (6) oder der mit der Abdeckung (6) verbundene Anschlussring (5) vom Behälter gelöst und der Montagering (4) erfasst und mit dem Absaugbeutel (3) aus dem Behälter (2) entfernt wird.

14. Verfahren nach Anspruch 13, **dadurch gekennzeichnet, dass** der Absaugbeutel (3) eine Zugleine (34) umfasst, die nach der Entfernung des Absaugbeutels (3) aus dem Behälter (2) betätigt wird, um die Beutelöffnung (30) abzuschliessen.

15. Verfahren nach Anspruch 13 oder 14, **dadurch gekennzeichnet, dass** der Montagering (4) vom gefüllten Absaugbeutel (3) entfernt und mit einem neuen Absaugbeutel (3) verbunden und der gefüllte Absaugbeutel (3) entsorgt wird oder dass der Montagering (4) mit dem gefüllten Absaugbeutel (3) entsorgt wird.
